# EUROPÄISCHE PATENTANMELDUNG

(11) **EP 1 902 685 A2**
(43) Veröffentlichungstag der Anmeldung: **26.03.2008**
(21) Anmeldenummer: 07018575.6
(22) Anmeldetag: 21.09.2007
(51) Int. Cl.: A61B 19/08

(54) **Operationstuch**

(30) Priorität: 22.09.2006 DE 202006014746 U
(71) Anmelder: Geuder AG, 69126 Heidelberg (DE)
(72) Erfinder: Geuder, Volker, 69126 Heidelberg (DE)
(74) Vertreter: Ullrich & Naumann

(57) **Zusammenfassung**

Ein Operationstuch (1) zur zumindest teilweisen Abdeckung des menschlichen oder tierischen Körpers bei einer medizinischen, vorzugsweise ophthalmologischen, Behandlung, mit einer Öffnung (2), wobei die Öffnung (2) mit einem flexiblen Flächenelement (3) verschlossen ist, ist dadurch gekennzeichnet, dass in dem flexiblen Flächenelement (3) ein Schlitz (5) vorgesehen ist, der entweder bereits voll ausgebildet oder im Sinne einer Sollbruchstelle vorbereitet ist, wobei die zu behandelnde Körperstelle durch den Schlitz (5) zugänglich ist.

## Beschreibung

Die Erfindung betrifft ein Operationstuch zur Abdeckung des menschlichen oder tierischen Körpers bei einer medizinischen, vorzugsweise ophthalmologischen, Behandlung mit einer Öffnung, wobei die Öffnung mit einem flexiblen Flächenelement verschlossen ist.

Operationstücher der in Rede stehenden Art sind seit Jahren aus der Praxis bekannt. Sie werden insbesondere bei chirurgischen Eingriffen dazu verwendet, im Laufe sowie unmittelbar nach der Behandlung das Eindringen von Krankheitserregern in die offene Wunde zu vermeiden, da diese aufgrund der einfachen Zugänglichkeit für Krankheitserreger eine hochempfindliche Stelle für Infektionen aller Art darstellt. Um die Risiken derartiger Infektionen zu minimieren, verwendet der Operateur zweckmäßigerweise ein aus einem flexiblen, zumeist atmungsaktiven Material beschaffenes Operationstuch, mit welchem er vor Durchführung der Behandlung den menschlichen oder tierischen Körper zumindest teilweise abdeckt.

Die Abdeckung bewirkt zum einen, dass Krankheitserreger wie Keime und Bakterien, die sich an der Haut oder den Haaren des Patienten abgesetzt haben, kaum in den Bereich der Wunde gelangen können, da die durch das Operationstuch abgedeckten Körperteile dann nicht in Kontakt mit dem Operateur, seinen Assistenten oder den Operationsinstrumenten gelangen können, was zu einer unerwünschten Übertragung in den empfindlichen Wundbereich führen könnte.

Um einen Zugang zu der zu behandelnden Körperstelle zu gewähren, sind die Operationstücher mit einer Öffnung versehen, welche die zu behandelnde Körperstelle in ausreichendem Maße freigibt. Um die Gefahr von Verunreinigungen weitestgehend zu verringern, kommt es bei der Ausführung der in dem Tuch vorgesehenen Öffnung darauf an, dass diese möglichst gut mit dem Wundrand abschließt. Da es jedoch schwierig oder gar unmöglich ist, bereits vor Beginn der Behandlung die genaue Form und Größe der benötigten Öffnung zu bestimmen, werden häufig Operationstücher mit einer vorgegebenen Öffnung verwendet, deren Öffnung zunächst mit einer zweckmäßigerweise transparenten Folie verschlossen ist.

So ist es möglich, den Patienten zunächst mit dem Operationstuch abzudecken und die Öffnung des Operationstuchs dabei über der zu behandelnden Körperstelle zu positionieren. Durch die transparente Ausführung der Folie ist die Positionierung deutlich erleichtert, da die zu behandelnde Körperstelle wie durch ein Fenster sichtbar ist. Um nach geeigneter Positionierung zur Durchführung der Behandlung Zugang zu der zu behandelnden Körperstelle zu erhalten, nimmt der Operateur zu Beginn der eigentlichen Behandlung einen Einschnitt in die Folie mit einem Messer vor. Durch die Notwendigkeit dieses Schnitts ergibt sich die Gefahr einer unnötigen Verletzung des Patienten. Des Weiteren kann der zusätzliche Schnitt beängstigend auf den Patienten wirken, was insbesondere in der Ophthalmologie von hoher Bedeutung ist, da hier die Eingriffe häufig lediglich unter Regional- oder Lokalanästhesie durchgeführt werden. Nicht zuletzt bedeutet der Schnitt einen zusätzlichen handwerklichen Aufwand für den Operateur, zu dem ein weiteres Werkzeug benötigt wird.

Der vorliegenden Erfindung liegt daher die Aufgabe zugrunde, ein Operationstuch der eingangs genannten Art derart auszugestalten und weiterzubilden, dass dieses bei Gewährleistung einer keimfreien Abdeckung der zu behandelnden Körperstelle ungefährlich und einfach zu handhaben ist.

Erfindungsgemäß wird die voranstehende Aufgabe durch die Merkmale des Patentanspruchs 1 gelöst. Danach ist das in Rede stehende Operationstuch dadurch gekennzeichnet, dass in dem flexiblen Flächenelement ein Schlitz vorgesehen ist, der entweder bereits voll ausgebildet oder im Sinne einer Sollbruchstelle vorbereitet ist, wobei die zu behandelnde Körperstelle durch den Schlitz zugänglich ist.

In erfindungsgemäßer Weise ist zunächst erkannt worden, dass es für den Operateur nachteilig ist, dass er zur Freigabe der Öffnung einen Schnitt in das flexible Flächenelement vornehmen muss. Für dieses erkannte Problem ist eine überraschend einfache Lösung gefunden worden, indem nämlich bei dem erfindungsgemäßen Operationstuch in dem flexiblen Flächenelement nunmehr bereits ein Schlitz vorgesehen ist, so dass dieser nicht mehr zu Anfang der Behandlung mittels eines extra vorzunehmende Schnitts erzeugt werden muss. Dies stellt eine erhebliche Arbeiterleichterung für den Operateur sowie Gefahrminderung für den Patienten dar.

Erfindungsgemäß ist es dabei denkbar, dass der Schlitz in dem flexiblen Flächenelement entweder bereits voll ausgebildet ist oder dass er im Sinne einer Sollbruchstelle vorbereitet ist. Im ersteren Fall lässt sich die medizinische Behandlung unmittelbar nach Positionierung des Operationstuchs beginnen, im letzteren Fall lässt sich ein Zugang zur behandelnden Körperstelle auf einfache Weise durch Öffnen des flexiblen Flächenelements an der vorbereiteten Sollbruchstelle verschaffen. Bei entsprechender Ausführung der Sollbruchstelle - beispielsweise durch Perforation des Flächenelements an der Sollbruchstelle - ist hierzu kein spezielles Werkzeug erforderlich, sondern lässt sich der Schlitz durch manuelles Aufreißen, Auseinanderziehen oder dergleichen erzeugen. Das erfindungsgemäße Operationstuch ermöglicht somit einen günstigen Schutz der zu behandelnden Körperstelle bei gleichzeitig deutlich vereinfachter Handhabung, da kein Werkzeug oder Instrument mehr benötigt wird.

Im Konkreten lässt sich das erfindungsgemäße Operationstuch derart ausgestalten, dass das flexible Flächenelement eine die Öffnung überdeckende Fläche aufweist. Durch diese Maßnahme entsteht in dem Bereich des Tuchs, welcher die Öffnung umgibt, eine Überlappung des flexiblen Flächenelements mit dem Tuch, so dass sich das flexible Flächenelement auf günstige Weise an dem Tuch fixieren lässt. Grundsätzlich ist es dabei denkbar, das flexible Flächenelement an der der zu behandelnden Körperstelle zugewandten oder abgewandten Seite des Tuchs anzuordnen. Bei einer Anordnung an der dem Körper abgewandten Seite des Tuchs ergibt sich der Vorteil, dass sich das flexible Flächenelement bei Bedarf komplett von dem Tuch ablösen lässt, da es von außen vollständig zugänglich ist.

Alternativ ergibt sich bei Anordnung des flexiblen Flächenelements an der dem Körper zugewandten Seite des Tuchs der besondere Vorteil, dass ein über die Größe der Öffnung hinausgehender Bereich des flexiblen Flächenelements an der zu behandelnden Körperstelle bzw. um die zu behandelnde Körperstelle herum verfügbar ist.

Die sich daraus ergebende zusätzliche Kontaktfläche zwischen dem flexiblen Flächenelement und den Hautpartien des Patienten kann dazu genutzt werden, eine mehr oder weniger großflächige Fixierung des Operationstuchs am Körper zu erzielen. Hierzu könnte an dem flexiblen Flächenelement körperseitig - also an der der zu behandelnden Körperstelle zugewandten Seite - ein klebender Bereich vorgesehen sein, der durch Haftung ein Verrutschen des flexiblen Flächenelements - und damit des Operationstuchs - wirksam verhindert. Dabei ist es denkbar, den klebenden Bereich im Wesentlichen am Randbereich des flexiblen Flächenelements, also vorzugsweise außerhalb der Öffnung, vorzusehen, um nämlich ein eventuell störendes Festkleben unmittelbar an der zu behandelnden Körperstelle zu vermeiden. Alternativ könnte sich der klebende Bereich über die gesamte körperseitige Fläche des flexiblen Flächenelements erstrecken, wodurch fertigungstechnisch eine Vereinfachung gegeben ist.

Grundsätzlich kann es sinnvoll sein, einen körperseitigen Bereich, oder sogar die gesamte Fläche, des flexiblen Flächenelements mit einer Schutzfolie abzudecken. Die Schutzfolie ist dabei zweckmäßigerweise entfernbar ausgeführt. Jedenfalls lassen sich die sterilen Eigenschaften des flexiblen Flächenelements durch diese Maßnahme bis unmittelbar vor Aufbringen des flexiblen Flächenelements auf die zu behandelnde Körperstelle aufrechterhalten. In weiterhin vorteilhafter Weise lässt sich hierdurch die gegebenenfalls vorgesehene Klebwirkung des flexiblen Flächenelements ebenfalls aufrechterhalten.

Eine einfache Herstellbarkeit von flexiblem Flächenelement sowie darauf aufgebrachter Schutzfolie kann in vorteilhafter Weise dadurch erreicht werden, dass auch in der Schutzfolie ein Schlitz ausgebildet oder im Sinne einer Sollbruchstelle vorbereitet ist, welcher sich mit dem im flexiblen Flächenelement vorgesehenen Schlitz deckt.

Grundsätzlich ist es denkbar, dass der in dem flexiblen Flächenelement und gegebenenfalls auch in der entfernbaren Schutzfolie vorgesehene Schlitz durch Falzung, Perforation und/oder dünne Ausführung des Materials vorbereitet ist. Durch derartige Maßnahmen ergibt sich jedenfalls eine Materialschwächung, durch welche die Sollbruchstelle auf einfache Weise vorgegeben sein könnte. Im Ergebnis führt diese Sollbruchstelle dazu, dass die Zugänglichkeit zu der zu behandelnden Körperstelle ohne Einsatz eines zusätzlichen Werkzeugs bzw. Instruments möglich ist. Andererseits bietet sich der Vorteil, dass die Öffnung bis kurz vor der Behandlung weitestgehend verschlossen bleibt.

Das flexible Flächenelement ist in weiterhin vorteilhafter Weise transparent ausgeführt, um die Sichtbarkeit der zu behandelnden Körperstelle sowie der angrenzenden Hautpartien zu gewährleisten. Störende optische Effekte können sich dadurch vermieden werden, dass das flexible Flächenelement zusätzlich reflexionsfrei ausgeführt ist. Es bietet sich somit an, das flexible Flächenelement als Folie auszuführen, wobei sich Kunststofffolien, insbesondere aus Polyurethan, in besonderer Weise zu diesem Zweck eignen.

Auf vorteilhafte Weise ist die Verbindung zwischen dem flexiblen Flächenelement und dem Tuch stoffschlüssig, wobei insbesondere durch Kleben eine Materialschonende, leicht herzustellende Verbindung ermöglicht ist.

Hinsicht der in dem Tuch ausgebildeten Öffnung ist es grundsätzlich denkbar, diese in beliebigen Formen auszubilden. Insbesondere im Einsatzgebiet der Ophthalmologie kann es von Vorteil sein, die Öffnung ovalförmig, vorzugsweise elliptisch, auszubilden, so dass sich eine im Wesentlichen dem Auge angepasste Form ergibt.

Der in dem flexiblen Flächenelement vorgesehene Schlitz könnte im Falle einer elliptischen Form der Öffnung in vorteilhafter Weise entlang oder parallel der Haupt- und/oder Nebenachse der Ellipse verlaufen. Durch einen derartigen Verlauf des Schlitzes wird beispielsweise das Einführen und Befestigen einer Lidsperre vereinfacht, wobei diese dann die sich am Schlitz gegenüberliegenden Flächenabschnitte des flexiblen Flächenelements auseinander zieht und somit die zu behandelnde Körperstelle - in diesem Falle den Augapfel - auf günstige Weise zugänglich macht. Bei einer anderen Form der Öffnung könnte der Schlitz zumindest entlang oder parallel der größten Ausdehnung der Öffnung verlaufen.

Alternativ kann der Schlitz auch auf beliebig andere Weise innerhalb eines sich mit der Öffnung deckenden Bereichs des flexiblen Flächenelements verlaufen. Dabei ist es vorteilhaft, dass der Schlitz stets mit Abstand zum Rand der Öffnung verläuft.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: eine schematische Draufsicht auf ein Operationstuch und
- Fig. 2: eine schematische Querschnittsansicht des Operationstuchs aus Fig. 1 entsprechend dem Schnitt A-A.

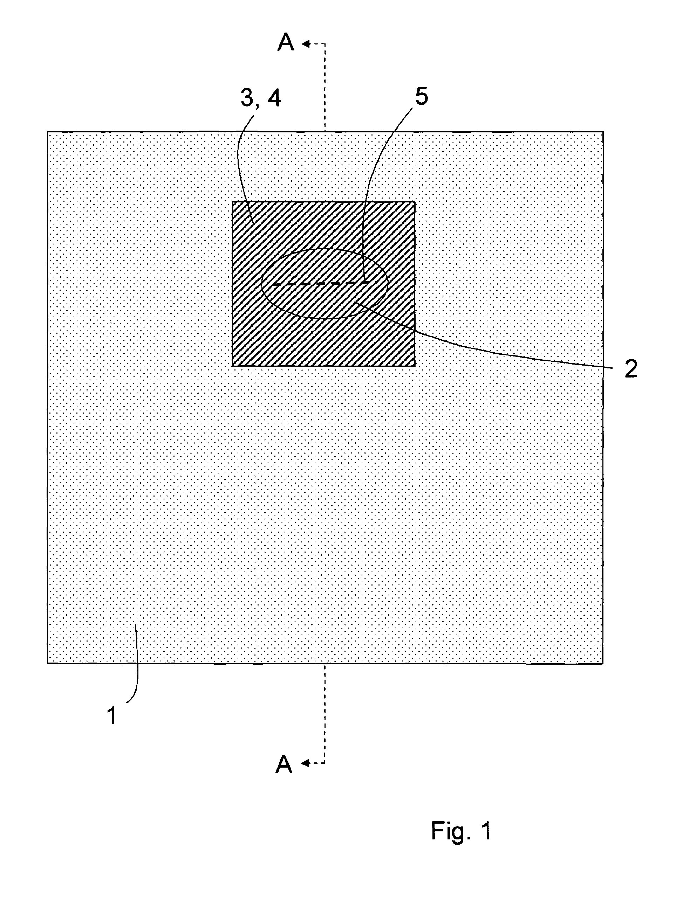

Das in Fig. 1 gezeigte Operationstuch 1 - im Folgenden Tuch 1 - weist eine rechteckige Grundform auf. Das Tuch 1 ist aus einem Vliesstoff gefertigt und eignet sich zur Abdeckung des menschlichen oder tierischen Körpers während einer medizinischen Behandlung. In einem oberen Bereich des Tuches 1 ist mittig eine Öffnung 2 mit elliptischer Grundform ausgebildet, durch welche die zu behandelnde Körperstelle letztlich zugänglich ist. Aufgrund der elliptischen Form der Öffnung 2 eignet sich das gezeigte Tuch 1 in besonderer Weise zur Anwendung bei ophthalmologischen Behandlungen.

Fig. 1 zeigt weiterhin, dass die Öffnung 2 mit einem flexiblen Flächenelement 3 verschlossen ist, welches eine rechteckige, die Öffnung 2 großzügig überdeckende Grundfläche aufweist. In den an die Öffnung 2 herum angrenzenden, überlappenden Flächenbereichen sind das Tuch 1 und das flexible Flächenelement 3 klebend miteinander verbunden. Da das flexible Flächenelement 3 eine transparente, reflexionsfreie Kunststofffolie ist, ist die zu behandelnde Körperstelle - die hier nicht dargestellt ist, sich in der Darstellung der Fig. 1 jedoch über dem Tuch 1 befindet - auch durch das flexible Flächenelement 3 hindurch sichtbar. Zur Fixierung des flexiblen Flächenelements 3 - und damit zur Fixierung des damit verbundenen Tuchs 1 - am Körper des Patienten ist die gesamte körperseitige Fläche des flexiblen Flächenelements 3 klebend ausgeführt. Neben der Fixierung wird hierdurch weiterhin die sterile Abdeckung des an die zu behandelnde Körperstelle angrenzenden Hautbereichs erzielt. Hierdurch wird u.a. das Wandern pathogener Hautkeime des Patienten zur Behandlungsstelle verhindert und somit die Infektionsgefahr für den Patienten verringert.
Um dem Operateur eine einfache Zugänglichkeit zu der zu behandelnden Körperstelle zu ermöglichen, ist bei dem in Fig. 1 gezeigten Tuch 1 in dem flexiblen Flächenelement 3 ein Schlitz 5 vorgesehen, der im Sinne einer Sollbruchstelle vorbereitet ist. Dieser Schlitz 5 verläuft entlang der Hauptachse der elliptischen Öffnung 2, beginnt und endet jedoch mit Abstand zum Rand der Öffnung 2. Wie durch die gestrichelte Linie angedeutet, ist der Schlitz 5 nicht bereits voll ausgebildet, sondern durch Perforation als eine Sollbruchstelle vorbereitet. Diese Perforation führt dazu, dass sich entlang dieser Linie aufgrund der durch die Perforation hervorgerufenen Materialschwächung durch leichtes Aufbringen eines Drucks beispielsweise durch den Operateur der Schlitz 5 in dem flexiblen Flächenelement 3 voll ausbildet.

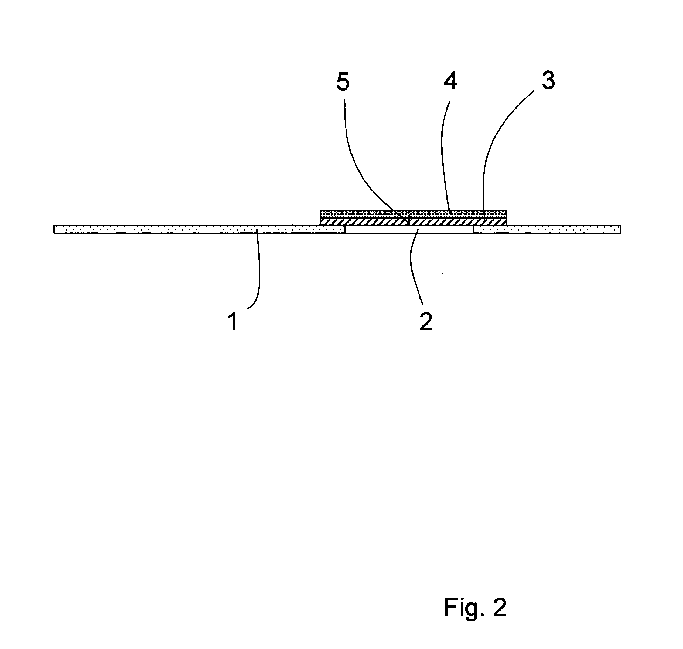

Zur Verhinderung von Verunreinigungen des klebenden Bereichs des flexiblen Flächenelements 3 vor der medizinischen Behandlung ist dieses mit einer Schutzfolie 4 abgedeckt. Dies ist am besten in Fig. 2, welche das Tuch 1 aus Fig. 1 gemäß dem Schnitt A-A zeigt, von der Seite zu sehen. Wie ebenfalls in Fig. 2 dargestellt, ist auch die Schutzfolie 4 mit einer Perforation versehen, welche deckungsgleich zu der im flexiblen Flächenelement 3 ausgebildeten Perforation verläuft.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Schutzansprüche verwiesen.

Schließlich sei ausdrücklich darauf hingewiesen, dass das voranstehend beschriebene Ausführungsbeispiel des erfindungsgemäßen Operationstuchs lediglich zur Erörterung der beanspruchten Lehre dient, dieses jedoch nicht auf das Ausführungsbeispiel einschränkt.

## Patentansprüche

1. Operationstuch (1) zur zumindest teilweisen Abdeckung des menschlichen oder tierischen Körpers bei einer medizinischen, vorzugsweise ophthalmologischen, Behandlung, mit einer Öffnung (2), wobei die Öffnung (2) mit einem flexiblen Flächenelement (3) verschlossen ist,
**dadurch gekennzeichnet, dass** in dem flexiblen Flächenelement (3) ein Schlitz (5) vorgesehen ist, der entweder bereits voll ausgebildet oder im Sinne einer Sollbruchstelle vorbereitet ist, wobei die zu behandelnde Körperstelle durch den Schlitz (5) zugänglich ist.

2. Operationstuch nach Anspruch 1, **dadurch gekennzeichnet, dass** das flexible Flächenelement (3) eine die Öffnung (2) überdeckende Fläche aufweist, wobei
das flexible Flächenelement (3) vorzugsweise an der Seite des Tuchs (1) angeordnet ist, die der zu behandelnden Körperstelle zugewandt ist.

3. Operationstuch nach Anspruch 1 oder 2, **dadurch gekennzeichnet, dass** das flexible Flächenelement (3) körperseitig einen klebenden Bereich aufweist, wobei
sich der klebende Bereich vorzugsweise über die gesamte körperseitige Fläche des flexiblen Flächenelements (3) erstreckt.

4. Operationstuch nach einem der Ansprüche 1 bis 3, **dadurch gekennzeichnet, dass** ein körperseitiger Bereich, vorzugsweise die gesamte körperseitige Fläche, des flexiblen Flächenelements (3) mit einer Schutzfolie (4) abgedeckt ist, wobei
in der Schutzfolie (4) ein Schlitz ausgebildet oder im Sinne einer Sollbruchstelle vorbereitet sein kann, der mit dem im flexiblen Flächenelement (3) vorgesehenen Schlitz (5) deckungsgleich ist.

5. Operationstuch nach einem der Ansprüche 1 bis 4, **dadurch gekennzeichnet, dass** das flexible Flächenelement (3) und gegebenenfalls die entfernbare Schutzfolie (4) an der Sollbruchstelle gefalzt, perforiert und/oder dünn ausgeführt ist bzw. sind.

6. Operationstuch nach einem der Ansprüche 1 bis 6, **dadurch gekennzeichnet, dass** das flexible Flächenelement (3) transparent ist und/oder
dass das flexible Flächenelement (3) reflexionsfrei ist und/oder
dass das flexible Flächenelement (3) eine Folie, vorzugsweise eine Kunststofffolie, ist.

7. Operationstuch nach einem der Ansprüche 1 bis 6, **dadurch gekennzeichnet, dass** das flexible Flächenelement (3) mit dem Tuch (1) stoffschlüssig, vorzugsweise durch Kleben, verbunden ist.

8. Operationstuch nach einem der Ansprüche 1 bis 7, **dadurch gekennzeichnet, dass** die Öffnung (2) ovalförmig, vorzugsweise elliptisch, ausgebildet ist, wobei
der Schlitz (5) im Wesentlichen entlang oder parallel der der größten Ausdehnung der Öffnung (2) verlaufen kann.

9. Operationstuch nach einem der Ansprüche 1 bis 8, **dadurch gekennzeichnet, dass** der Schlitz (5) innerhalb eines Bereichs des flexiblen Flächenelements (5) verläuft, welcher sich mit der Öffnung (2) deckt.

10. Operationstuch nach Anspruch 9, **dadurch gekennzeichnet, dass** der Schlitz (5) mit Abstand zum Rand der Öffnung (2) verläuft.
